# EUROPEAN PATENT APPLICATION

(11) **EP 2 218 445 A1**
(43) Date of publication of application: **18.08.2010**
(21) Application number: 10153272.9
(22) Date of filing: 11.02.2010
(51) Int. Cl.: A61K 9/48, A61K 9/50

(54) **A microparticle containing a microorganism or biological material, and a process for producing the same**

(30) Priority: 13.02.2009 JP 2009031696
(71) Applicant: Freund Corporation, Tokyo 163-6034 (JP)
(72) Inventor: Yasumi, Hirotsune, Tokyo 163-6034 (JP); Sugiyama, Mamoru, Tokyo 163-6034 (JP); Kusanagi, Koichi, Tokyo (JP); Hosokawa, Tomoko, Tokyo (JP); Takeyama, Natsumi, Tokyo (JP); Sato, Tetsuo, Tokyo (JP)
(74) Representative: Raggers, René John

(57) **Abstract**

Provided is a microparticle containing a microorganism or biological material with an excellent preservation stability. A microparticle containing a microorganism or biological material having a three-layered structure composed of an outermost shell layer, an intermediate layer, and a core, wherein the outermost shell layer is composed of a composition containing gelatin and natural gum, an aqueous solution of the composition has a gelling temperature of 60°C or lower, the intermediate layer is composed of a hydrophobic substance having a viscosity at 40°C of 250 cps or less and a viscosity at 5°C of 300 cps or more, the core is composed of a composition containing a microorganism or biological material, and a water content of the whole particle is 5% or less, and a water activity is 0.5 or less.

## Description

### BACKGROUND OF THE INVENTION

### Field of the Invention

Priority is claimed on Japanese Patent Application No. 2009-031696, filed February 13, 2009, the content of which is incorporated herein by reference.

The present invention relates to a microparticle containing a microorganism or biological material, which can be suitably used, for example, in an oral vaccine for animals, and a process for producing the same.

### Description of Related Art

In general, pharmaceutical drugs, such as vaccines containing a microorganism or biological material, are usually administered to a subject animal by injection. Disadvantageously, however, the injection procedure is not simple, and the inoculum is liable to leave residue at the injection site, or side effects such as fever, flare, muscular atrophy, and shock are liable to be caused. Particularly in the case where the subject is an industrial animal such as a cow, a pig, or a bird, the labor of injection is enormous for immunizing a large number of animals in a short period of time. Therefore, oral administration is desirable for large scale immunization of animals.

In real practices, only a few kinds f oral vaccines have been effectively used, and the vaccines that have been used are merely products in which a microorganism or biological material which is resistant to acids and proteases and whose action site is located before the stomach where they are exposed to acids or the like, or products which are not susceptible to the influence of moisture, specifically poliomyelitis vaccines and Newcastle disease vaccines. A reason for this is that most of microorganisms or biological material in oral vaccines are inactivated by the action of moisture during preservation and storage, or such substances, due to high sensitivity to gastric acid or proteases, are inactivated by the action of acids or proteolytic enzymes in the stomach during delivery to a mucosal surface of the intestinal tract where the vaccine exerts its efficacy following oral administration.

As an effective approach to cope with the above-mentioned problems, there is a method in which the water content of the whole capsule is reduced to a minimum so as to prevent inactivation of the microorganism or the biological material by the action of moisture upon preservation thereof, or a method in which the microorganism or the biological material is encapsulated by a capsule (intestine-soluble capsule) which is not digested in the stomach but is soluble in the intestine, followed by administration, when the microorganism or the biological material exhibits high sensitivity to gastric acid.

The present inventors have previously published a process for producing an intestine-soluble particle having a core layer containing an immunogen, and one or plural shell layers for covering the core layer, the outermost layer being composed of an intestine-soluble material, using a concentric multiple nozzle (Japanese Unexamined Patent Application Publication No. H10-130166). Specifically, what was provided is a process for producing an intestine-soluble particle, including extruding a suspension of an immunogen from a center tube of a concentric multiple nozzle, and simultaneously extruding a solution of an intestine-soluble material from a outermost tube of the concentric multiple nozzle to allow the extruded matter to drop into a bath for solidification thereof.

Still, depending on production conditions, the quality of the product produced during a prolonged time is unstable, and when an immunogen is liable to be inactivated by water contained in the particle, the storage period of the intestine-soluble particle without efficacy reduction is severely shortened. To this end, there is a need for improvements in the preservation stability.

Japanese Unexamined Patent Application Publication No. H8-10313 discloses a seamless capsule wherein charged aqueous droplets are covered with a gelatin-based shell material where the droplet and the outer shell are separated by an intermediate layer. An advantage of such a capsule is its ability to hold a large volume of a charged aqueous liquid in a capsule, wherein only the water in the gelatin-based outer shell is removed while maintaining the core droplets in an aqueous liquid state, and the final form maintains water in the capsule.

Japanese Unexamined Patent Application Publication No. S62-263128 discloses a seamless capsule wherein beneficial enterobacteria are separated from an outer coating of the capsule with a hydrophobic substance which is non-fluid at room temperature. A core liquid is a dispersion of beneficial enterobacteria in non-aqueous glycerin, non-fluid hydrophobic substance or the like, and not in an aqueous liquid. Further, an intermediate layer is composed of Japan wax which is a non-fluid hydrophobic substance.

### SUMMARY OF THE INVENTION

Therefore, the present invention has been made in view of the above problems, and it is an object of the present invention to provide a microparticle containing a microorganism or biological material with superior preservation stability, and a process for producing the same.

In order to overcome the above-mentioned problems, the microparticle containing a microorganism or biological material is provided. The particle has a three-layered structure composed of an outermost shell layer, an intermediate layer, and a core, wherein the outermost shell layer is composed of a composition containing gelatin and natural gum, a aqueous solution of the composition has a gelling temperature of 60°C or lower, the intermediate layer is composed of a hydrophobic substance having a viscosity at 40°C of 250 cps or less and a viscosity at 5°C of 300 cps or more, the core is composed of a composition containing a microorganism or biological material, and a water content of the whole particle after drying is 5% or less and a water activity is 0.5 or less.

The microparticle containing a microorganism or biological material preferably has a network structure which is formed by the reaction of divalent cations in a reaction solution with natural gum in the outermost shell layer.

The hydrophobic substance constituting the intermediate layer is preferably a mixture of lecithin and lipids.

A process for producing the microparticle containing a microorganism or biological material of the present invention includes forming a seamless capsule precursor having a three-layered structure composed of the outermost shell layer, the intermediate layer, and the core liquid, by a method of extrusion into a solidifying liquid using a concentric triple nozzle, and drying the seamless capsule precursor to a water content of the whole particle of 5% or less and a water activity of 0.5 or less, wherein the outermost shell layer is composed of a composition containing gelatin and natural gum, an aqueous solution of the composition has a gelling temperature of 60°C or lower, the intermediate layer is composed of a hydrophobic substance having a viscosity at 40°C of 250 cps or less and a viscosity at 5°C of 300 cps or more, and the core liquid is composed of an aqueous liquid containing a microorganism or biological material.

The process preferably includes a step of dipping the seamless capsule precursor extruded from the concentric triple nozzle into a liquid containing divalent cations, prior to drying.

Drying of the seamless capsule precursor is preferably carried out at a temperature equal to or higher than the temperature where the intermediate layer has a viscosity of 250 cps or less and at a drying temperature equal to or lower than the gelling temperature of an aqueous solution of the composition constituting the outermost shell layer.

A center tube and/or an intermediate tube of the concentric triple nozzle preferably have thermal insulation properties.

In accordance with the present invention, a microparticle containing a microorganism or biological material with an excellent stability can be obtained.

In cases a microorganism or biological material exhibits high sensitivity to gastric acid, the microparticle containing a microorganism or biological material with a network structure at the outmost layer can be used. The particles with the network structure are produced by dipping a seamless capsule precursor extruded from the concentric triple nozzle into a liquid containing divalent cations, prior to drying, leading to formation of microparticles with enteric properties after drying.

### BRIEF DESCRIPTION OF THE DRAWINGS

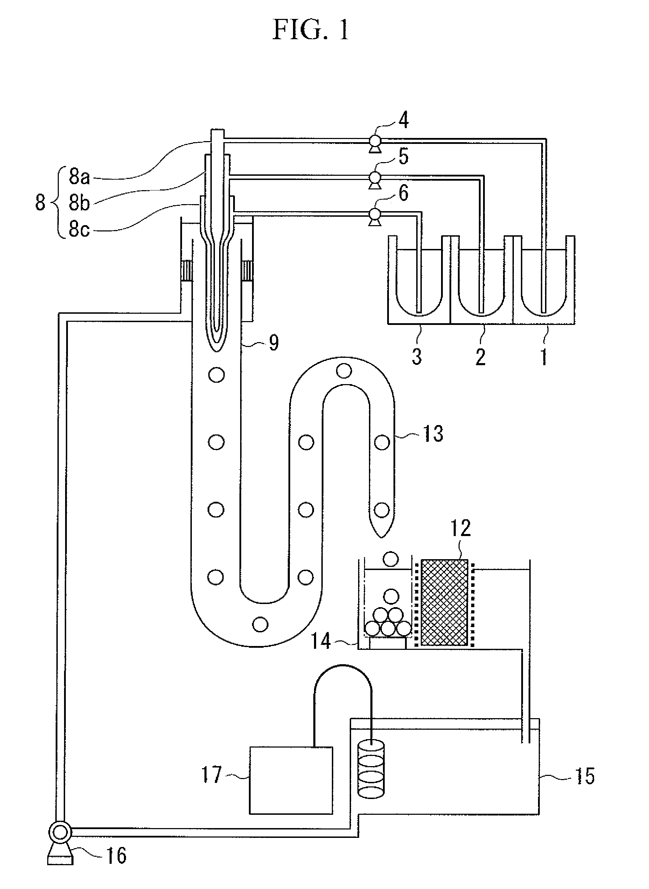

FIG. 1 is a schematic diagram showing an example of an apparatus which is suitable for the production of a microparticle containing a microorganism or biological material relating to the present invention.

### DETAILED DESCRIPTION OF THE INVENTION

### Composition containing a microorganism or biological material (core, core liquid)

The core is composed of a composition containing a microorganism or biological material. The core is formed by drying a core liquid consisting of an aqueous liquid of the composition.

In the present invention, the aqueous liquid of a composition containing a microorganism or biological material is used as the core liquid, when a seamless capsule precursor having a three-layered structure is produced using a concentric triple nozzle. For example, the aqueous liquid (core liquid) is a vaccine containing viruses or bacteria or components thereof (including peptides) as an immunogen, or an aqueous liquid in which a microorganism or biological material is dissolved or dispersed. Further, a recombinant vaccine, DNA vaccine, or the like may also be used, even though a system of classification may be different. Further, the composition may contain stabilizers such as gelatin and lactose, colorants, and the like, as components other than a microorganism or biological material.

For example, when an aqueous liquid containing living viruses such as attenuated strains is used as the core liquid, it is important to prevent the inactivation of viruses throughout the production process and the application steps. Avoiding exposure of the pathogen to high temperatures, organic solvents, high humidity (such as high water activity value due to a high water content) for a long period of time, and extremely high or low pH are the basic universal conditions for prevention of inactivation, although the required specific conditions may vary depending on the respective viruses.

Specific examples of a microorganism or biological material that can be used in the present invention include viruses or bacteria such as influenza, Japanese encephalitis, pertussis, cholera, pneumococcus, Weil's disease, dysentery, typhoid fever, paratyphoid fever, typhus fever, scarlet fever, epidemic encephalomyelitis, plague, Lassa fever, malaria, yellow fever, anthrax, infectious diarrhea, tsutsugamushi disease, filarial disease, relapsing fever, schistosomiasis, trachoma, syphilis, gonorrhea, chancroid, lymphogranuloma inguinale, Hansen's disease, Newcastle disease, infectious coryza, avian infectious bronchitis, avian infectious bursal disease, Mycoplasma gallisepticum infection, fowl cholera, fowl plague, pullorum disease, duck plague, rabbit viral hemorrhagic disease, porcine bordetellosis, porcine Actinobacillus infection, porcine epidemic diarrhea, porcine rotavirus infection, porcine Aujeszky's disease, transmissible gastroenteritis of swine, porcine adenovirus infection, porcine reproductive and respiratory syndrome (PRRS), porcine parvovirus infection, Glasser's disease, African swine fever, swine vesicular disease, vesicular stomatitis, swine dysentery, equine influenza, equine rhinopneumonitis, equine Getah virus infection, equine viral arteritis, glanders, equine infectious anemia, pseudofarcy, equine paratyphoid, African horse sickness, viral equine encephalitis, foot-and-mouth disease, cattle plague, bovine ephemeral fever, Ibaraki disease, Chuzan disease, Akabane disease, infectious bovine rhinotracheitis, bovine viral diarrhea, bovine parainfluenza infection, bovine adenovirus infection, bovine rotavirus infection, contagious bovine pleuropneumonia, blackleg, bovine hemorrhagic anemia, brucellosis, Johne's disease, piroplasmosis, anaplasmosis, blue tongue disease, Rift Valley fever, malignant catarrhal fever, heartwater, sheep pox, Maedi-Visna disease, contagious caprine pleuropneumonia, feline calicivirus infection, feline viral rhinotracheitis, feline panleukopenia, rabies, distemper, canine infectious hepatitis, canine infectious laryngotracheitis, canine parainfluenza infection, canine parvovirus infection, canine coronavirus infection, canine herpesvirus infection, canine oral papilloma, feline infectious peritonitis, feline leukemia, vibriosis of fishes, furunculosis, poliomyelitis, measles, rubella, tuberculosis (BCG), typhus, variola, epidemic parotitis encephalitis, avian infectious laryngotracheitis, fowlpox, Marek's disease, avian encephalomyelitis, hog cholera, swine erysipelas, diphtheria, tetanus, Habu toxoid, botulinus, hepatitis B, hepatitis C, herpes, bifidobacteria, and lactic acid bacillus; cytokines, hormones and the like.

### Hydrophobic substance (intermediate layer)

When it is desired to produce a seamless capsule precursor having a three-layered structure using a concentric triple nozzle, a hydrophobic substance having a viscosity at 40°C of 250 cps or less and a viscosity at 5°C of 300 cps or more is used as an intermediate layer. As used herein, the term "hydrophobic substance" refers to a "water-insoluble oily substance".

If a hydrophobic substance constituting the intermediate layer has a viscosity at 40°C of more 250 cps, this results in difficulty in transferring of water from the core liquid to the outermost shell layer, due to the presence of a hydrophobic substance having poor fluidity between the core liquid and the outermost shell layer, when drying the seamless capsule precursor. As a result, the removal of water from the core liquid is insufficient, whereby the residual water contributes to inactivation of a microorganism or biological material. Therefore, a viscosity of the hydrophobic substance at 40°C is preferably 200 cps or less, and a lower limit thereof is preferably 30 cps or above.

If a hydrophobic substance constituting the intermediate layer has a viscosity at 5°C of less 300 cps, this may result in no formation of a favorable three-layered structure because an intermediate layer (layer of hydrophobic substance) formed between the outermost shell layer (aqueous solution) and the core liquid (aqueous liquid) becomes readily fluid when extruding individual components from a concentric triple nozzle into a solidifying liquid to form a seamless capsule precursor. Therefore, the viscosity of the hydrophobic substance at 5°C is preferably in the range of 300 to 500 cps.

Such a hydrophobic substance is preferably a mixture of lecithin and lipids, particularly preferably a mixture of lecithin and medium-chain fatty acid triglyceride (MCT). A mixing ratio (mass ratio) of lecithin to MCT in the mixture of lecithin and MCT is preferably in the range of 1:9 to 7:3, and more preferably 3:7 to 7:3.

### Composition containing gelatin and natural gum (outermost shell layer)

The outermost shell layer is composed of a composition containing gelatin and natural gum. The outermost shell layer is formed using an aqueous solution (outermost shell layer liquid) of the composition.

A gelling temperature of the outermost shell layer is 60°C or lower. When a seamless capsule precursor is prepared using a concentric triple nozzle, the outermost shell layer liquid is used at a temperature higher than the gelling temperature thereof. Therefore, if a gelling temperature of the outermost shell layer liquid is higher than 60°C, the outermost tube of the concentric triple nozzle is warmed, which consequently brings about warming of the intermediate layer and the core liquid, whereby a microorganism or biological material contained in the core liquid becomes susceptible to degeneration (inactivation). A gelling temperature of the outermost shell layer liquid is preferably 50°C or lower.

Natural gum used herein has a gelling temperature of 60°C or lower, and is preferably one or more selected from the group consisting of gellan gum, carrageenan, xanthane gum, and gum arabic. Among these natural gums, particularly preferred is gellan gum as it reacts with divalent cations to form a network structure, and exhibits easy gellability. The mixing ratio (mass ratio) of gelatin to natural gum is preferably in the range of 25:0.1 to 10:2.0, and more preferably in the range of 8:0.1 to 8:0.5.

The composition containing gelatin and natural gum may contain a plasticizer such as glycerin, propylene glycol or sorbitol, as another component in addition to gelatin and natural gum. The total amount of other components in the composition is preferably equal to or less than that of gelatin. If the amount of other components is excessively high relative to gelatin, the outermost shell layer becomes too soft. On the other hand, if the amount of other components is excessively low relative to gelatin, the outermost shell layer becomes too rigid, and contributes to cracking. For these reasons, the amount of other components is more preferably 20.0 to 40.0% by mass of the gelatin content.

The outermost shell layer liquid preferably has a pH of 4.0 to 9.0, and more preferably has a pH of 5.0 to 8.0. When the pH of the outermost shell layer liquid is outside the above-specified range, a microorganism or biological material is susceptible to degeneration (inactivation).

### Liquid containing divalent cations (reaction liquid)

A liquid containing divalent cations is used as a reaction liquid for intestine-solubilization of the seamless capsule precursor extruded from the concentric triple nozzle.

Examples of the divalent cations may include calcium ions, magnesium ions, and the like. Among these divalent cations, calcium ions are preferred in terms of the reactivity.

The liquid containing divalent cations may be, for example, an aqueous solution of calcium lactate, an aqueous solution of calcium citrate, an aqueous solution of magnesium chloride, or the like. Among them, the aqueous solution of calcium lactate is preferred in terms of no change in pH, and the like.

### Production process

Hereinafter, preferred embodiments of a process for producing a microparticle containing a microorganism or biological material of the present invention will be described.

### Formation of a seamless capsule precursor having a three-layered structure

Firstly, a seamless capsule precursor having a three-layered structure composed of an outermost shell layer, an intermediate layer and a core liquid (hereinafter, referred to also as "multiplex capsule precursor") is prepared according to a method of extrusion in a solidifying liquid using a concentric triple nozzle.

FIG. 1 schematically shows an example of an apparatus equipped with a triple nozzle which is suitably used in the present invention. Reference numeral 8 in FIG. 1 represents a concentric triple nozzle (hereinafter, referred to simply as "nozzle"). Using this apparatus, a multiplex capsule precursor is prepared as follows.
(1) An aqueous liquid (core liquid) of a microorganism or biological material is delivered from a tank 1 by a pump 4 to a center tube 8a of the nozzle 8. A liquid-like hydrophobic substance (intermediate layer liquid) is delivered from a tank 2 by pump 5 to an intermediate tube 8b of the nozzle 8. An aqueous solution (outermost shell layer liquid) of a composition containing gelatin and natural gum is delivered from tank 3 by a pump 6 to the outermost tube 8c of the nozzle 8. A discharge port of the center tube 8a, a discharge port of the intermediate tube 8b, and a discharge port of the outermost tube 8c are provided in a concentric arrangement.
(2) The respective solutions sent to the nozzle 8 are extruded in a formation tube 9 from discharge ports of respective tubes. That is, the core liquid is extruded from the center tube, the intermediate layer liquid is extruded from the intermediate tube, and the outermost shell layer liquid is extruded from the outermost tube.

In the formation tube 9, a solidifying liquid is continuously fed from the upside by overflow by means of a pump 16, and the liquid discharge ports of the nozzle 8 are immersed in the solidifying liquid.
(3) The liquid extruded from the liquid discharge ports forms "jet" in the solidifying liquid in the formation tube, and the "jet" forms, owing to "surface tension", droplets in which the core liquid is enclosed by the intermediate layer liquid and further by the outermost shell layer liquid.

Temperature (hereinafter, also referred to as "nozzle outlet temperature") conditions of the respective core liquid, intermediate layer liquid and outermost shell layer liquid immediately prior to the extrusion thereof in the solidifying liquid are preferably in the following range.

If a nozzle outlet temperature of the core liquid is excessively high, this may result in degeneration (inactivation) of the microorganism or the biological material contained in the core liquid. Therefore, a nozzle outlet temperature of the core liquid is preferably equal to or lower than a nozzle outlet temperature of the intermediate layer liquid and more preferably equal to or lower than room temperature.

If a nozzle outlet temperature of the intermediate layer liquid is excessively high, the core liquid nozzle is warmed, which may result in degeneration (inactivation) of the microorganism or the biological material. On the other hand, if a nozzle outlet temperature of the intermediate layer liquid is excessively low, the viscosity of the intermediate layer liquid is high, thus leading to instability of the capsule precursor formation. Therefore, a nozzle outlet temperature of the intermediate layer liquid is preferably in the range of 20 to 40°C, and more preferably room temperature.

If a nozzle outlet temperature of the outermost shell layer liquid is excessively low, this results in blockage of the nozzle. Therefore, a nozzle outlet temperature of the outermost shell layer liquid is preferably in the range of 60 to 95°C, and more preferably 70 to 85°C.

The nozzle is usually made of stainless steel, and the stainless steel has a high heat conductivity at 300K of 15 to 20 W/(m·k). Therefore, the intermediate tube and the center tube are warmed by the outermost tube which was warmed by the outermost shell layer liquid, and consequently the core liquid is susceptible to temperature elevation.

In order to suppress the elevation of a core liquid temperature, it is preferred that materials consisting of the center tube 8a and/or the intermediate tube 8b have thermal insulation properties. Specifically, the center tube 8a and/or intermediate tube 8b are preferably made of a material having a heat conductivity at 300K of 5 W/(m.k) or less, and more preferably 2.5 W/(m.k) or less. Examples of such a material include, but are not limited to, resins such as polyetheretherketone, polyphenylene sulfide, polyamide, ethylene tetrafluoride, and polycarbonate, ceramics, and the like.

Examples of the solidifying liquid may include liquid paraffin, vegetable oils such as canola oil and MCT, silicone oil, and the like.

A temperature of the solidifying liquid is preferably in the range of 5 to 25°C, and more preferably 7 to 20°C, in order to prevent insufficient gelation of the outermost shell layer liquid.
(4) The droplets are cooled while being transported by the flow of the solidifying liquid and are solidified to become a multiplex capsule precursor. The solidified multiplex capsule precursor is delivered to a cooling liquid volume-adjusting tube 13 and then allowed to drop into a collecting device 14 to be separated from the solidifying liquid.
(5) The solidifying liquid separated from the multiplex capsule precursor is treated by a dehydration device 12 to remove the water, sent to a solidifying liquid tank 15, cooled to a given temperature by a heat exchanger 17, and then recycled to the formation tube 9 by a solidifying liquid pump 16.

The thus formed multiplex capsule precursor, when it is cooled by the solidifying liquid, exhibits the state where the intermediate layer is cooled to have high viscosity and semi-fluidity, and the outermost shell layer is cooled and then gelated to solidification.

### Dipping

Next, when the obtained multiplex capsule precursor is processed to have enteric properties, the precursor is dipped into a liquid (reaction liquid) containing divalent cations. In this manner, natural gum contained in the outermost shell layer reacts with divalent cations to form a network structure, followed by drying to obtain a multiplex capsule having enteric properties.

If a temperature of the reaction liquid is excessively high, the multiplex capsule precursor solidified by gelation dissolves. For this reason, a temperature of the reaction liquid is preferably equal to or lower than a temperature of the solidifying liquid.

Therefore, a temperature of the reaction liquid is preferably in the range of 5 to 25°C, and more preferably 7 to 20°C.

Further, enteric properties are affected by a degree of the reaction between the natural gum and the divalent cations. If a dipping time (reaction time) is excessively long, the resulting microparticle containing a microorganism or biological material becomes insoluble. On the other hand, if a dipping time is excessively short, sufficient enteric properties cannot be obtained. Therefore, a dipping time is preferably shorter than or equal to 20 minutes, and more preferably 3 to 10 minutes. When enteric properties are not required, it is not necessary to carry out the dipping process.

### Drying

Next, the multiplex capsule precursor is dried to obtain a particle composed of a seamless capsule. In the multiplex capsule precursor, the outermost shell layer and the core liquid contain water, so a hydrophobic substance constituting the intermediate layer is semi-fluid at a low temperature, and the fluidity thereof increases as the temperature increases. Therefore, water can be removed from the outermost shell layer and the core liquid layer by drying the capsule precursor at a temperature where a hydrophobic substance constituting the intermediate layer exhibits fluidity and at a temperature where the outermost shell layer is gelated to solidification. That is, if a drying temperature is excessively high, gelation of the outermost shell layer of the multiplex capsule precursor is destroyed to result in dissolution of the multiplex capsule. If a drying temperature is excessively low, this leads to a state where a viscosity of the intermediate layer is high and the core liquid is covered by a semi-fluid intermediate layer, whereby only the water in the outermost shell layer is removed, thus providing insufficient removal of water from the core liquid. Therefore, a drying temperature is preferably equal to or higher than a temperature at which the viscosity of the intermediate layer is 250 cps or less, and equal to or lower than a gelling temperature of the outermost shell layer. Specifically, a drying temperature is preferably in the range of 10 to 40°C, and more preferably in the range of 20 to 30°C. A more preferred drying temperature is equal to or higher than the temperature at which a viscosity of the intermediate layer is 200 cps or less, and equal to or lower than a gelling temperature of the outermost shell layer.

Although there is no particular limit to a drying method, air-drying, static drying, vacuum drying, or the like may be employed. Air-drying is preferred in terms of easy applicability to mass production.

The air-drying is a method of contacting the multiplex capsule with dry air controlled to the above-specified drying temperature. As used herein, the term "dry air" refers to air with relative humidity (RH) of 50% or less.

The air-drying is preferably carried out using a drum-type ventilation drying apparatus equipped with a rotary drum having a circumferential surface that can be ventilated, and a means which supplies dry air into the rotary drum and exhausts the used air to the outside through the circumferential surface of the rotary drum. In the drum-type ventilation drying apparatus, the multiplex capsule precursor is placed inside the rotary drum, and dry air is supplied to and exhausted from the drum while rotating the drum such that the multiplex capsule precursor flows in the rotary drum to contact dry air to remove water from the multiplex capsule precursor. Therefore, such a type of air-drying is preferable for the high-efficient production of a microparticle containing a microorganism or biological material because drying of the multiplex capsule precursor is efficiently carried out.

The drying conditions are set up such that the water content of the whole particle after drying is 5% or less, and a water activity is 0.5 or less. If the water content and the water activity of the whole particle are within the above-specified range, it is satisfactorily possible to prevent a decrease in an activity of a microorganism or biological material by the action of water. A more preferable water content value is 3% or less, and a more preferable water activity value is 0.4 or less.
In the present specification, the water content value of the whole particle is a value measured according to a dry weight loss test method in general test methods of the Japanese Pharmacopoeia 15^{th} edition, and the water activity value is a value measured by a water activity meter (product name: Pawkit, manufactured by INEX).

The particles dipped in divalent cations prior to drying thereof have enteric properties after drying, and can therefore be used as an intestine-soluble microparticle containing a microorganism or biological material which contains a microorganism or biological material functioning in the intestinal tract. As used herein, the term "particle having enteric properties" (an intestine-soluble microparticle containing a microorganism or biological material) refers to a particle which retains a particle shape without disruption even after being dipped in an artificial gastric juice for one hour.

The microparticle containing a microorganism or biological material has a post-drying particle diameter preferably ranging from 0.2 to 8 mm, and more preferably ranging from 0.5 to 5 mm. If the particle diameter is larger than 8 mm, it tends to be increasingly unsuitable for oral administration, and a microorganism or biological material functioning in the intestinal tract is susceptible to inactivation due to an excessively prolonged retention time in the stomach. On the other hand, if the particle diameter is less than 0.2 mm, it is difficult to secure a sufficient resistance to the gastric juice because of a larger surface area relative to a volume of the intestine-soluble microparticle containing a microorganism or biological material.

Since a microparticle containing a microorganism or biological material obtainable by a method of the present embodiment has an intermediate layer formed of a hydrophobic substance, the core liquid can be in the form of an aqueous liquid.
Because the hydrophobic substance has physical properties of 250 cps or less at 40°C, and 300 cps or more at 5°C, the intermediate layer can be fluidized even at a drying temperature which is low to the extent that it does not result in inactivation of a microorganism or biological material, so it is also possible to remove water from the core liquid inside the intermediate layer while preventing thermal inactivation. In this manner, adverse effects of water on a microorganism or biological material can be prevented, and therefore a superior preservation stability of the microparticle containing a microorganism or biological material can be obtained.

### Examples

The following Examples further illustrate the present invention in detail but are not to be construed as limiting the scope thereof. Hereinafter, "%" is "mass%" unless otherwise specifically indicated. (Examples 1 to 4)

Using an apparatus as shown in FIG. 1, a multiplex capsule precursor was prepared according to a method of the present invention. Composition formulas and production conditions of respective liquids are set forth in Table 1 below.

As a vaccine component, an aqueous solution of an attenuated virus "h-5 strain" derived from a virus (having a lipid membrane) of transmissible gastroenteritis of swine (TGE) was used as a microorganism or biological material which is susceptible to water content and water activity values upon preservation, and is also highly sensitive to gastric acid. This aqueous solution contains a stabilizer (10% lactose) and other additives. This attenuated virus, h-5 strain, is protease-sensitive, and its infection site is dislocated to the respiratory tract. This virus will be inactivated normally under environments of the stomach, and therefore cannot replicate in the small intestine. The h-5 strain is selected as a suitable material to evaluate whether enteric properties can be obtained. Naturally the present invention is not limited thereto. The virulent TGE virus infects the small intestine of infant swine to cause severe watery diarrhea to kill most of the swine which is seven-days-old or younger.

First, a core liquid, an intermediate layer liquid, and the outermost shell layer liquid, each having a composition as shown in Table 1, were extruded at a constant rate from the concentric triple nozzle 8 into a solidifying liquid to prepare multiplex capsule precursors. The temperatures and extrusion rates (liquid rates) of the core liquid, the intermediate layer liquid, the outermost shell layer liquid and the solidifying liquid are given in Table 1. As the solidifying liquid, Coconad MT (medium-chain fatty acid triglyceride, manufactured by Kao Corporation) was used.

For Example 1 and Comparative Example 1, the resulting multiplex capsule precursors were dried.

For Examples 2 and 3, and Comparative Example 2, the resulting multiplex capsule precursors were dipped in an aqueous calcium lactate solution (reaction liquid), and then dried. The composition, temperature, and dipping time (reaction time) of the reaction liquid are given in Table 1.

Drying was carried out to obtain dried multiplex capsules, using an innerduct type of drum-type ventilation drying apparatus, under the conditions as shown in Table 1. Any of the drying multiplex capsules obtained after drying gave a particle diameter within the range of 1 to 2 mm, and all of Examples 2 and 3 and Comparative Example 2 exhibited enteric properties.

**Table 1**

| | | Ex. 1 | Comp. Ex. 1 | Ex. 2 | Ex. 3 | Comp. Ex. 2 |
|---|---|---|---|---|---|---|
| Formulas | Core liquid formula | | | | | |
| | Gelatin | 5.5% | 5.5% | 5.5% | 5.5% | 5.5% |
| | Vaccine component | 65.5% | 65.5% | 65.5% | 65.5% | 65.5% |
| | Purified water | 29.0% | 29.0% | 29.0% | 29.0% | 29.0% |
| | Intermediate layer liquid formula | | | | | |
| | Lecithin | 50.0% | 50.0% | 40.0% | 50.0% | 60.0% |
| | MCT | 50.0% | 50.0% | 60.0% | 50.0% | 40.0% |
| | Outermost shell layer liquid formula | | | | | |
| | Gelatin | 15.0% | 15.0% | 15.0% | 15.0% | 15.0% |
| | Gellan gum | 0.5% | 0.8% | 0.5% | 0.8% | 0.8% |
| | Glycerin | 4.5% | 4.5% | 4.5% | 4.5% | 4.5% |
| | Purified water | 80.0% | 79.7% | 80.0% | 79.7% | 79.7% |
| Physical properties | Viscosity of intermediate layer liquid | | | | | |
| | 40°C | 55cps | 55cps | 50cps | 55cps | 60cps |
| | 5°C | 370cps | 370cps | 320cps | 370cps | 380cps |
| | Outermost shell layer liquid | | | | | |
| | Gelling temperature | 42°C | 45°C | 42°C | 45°C | 45°C |
| Reaction liquid | Formula | | | | | |
| | Calcium lactate | | | 0.5% | 1.0% | 1.0% |
| | Purified water | | | 99.5% | 99.0% | 99.0% |
| | Reaction time | | | 5min | 5min | 5min |
| Production conditions | Liquid temperature | | | | | |
| | Core liquid | 35°C | 35°C | 35°C | 35°C | 35°C |
| | Intermediate layer liquid | 25°C | 25°C | 25°C | 25°C | 25°C |
| | Outermost shell layer liquid | 85°C | 85°C | 85°C | 85°C | 85°C |
| | Solidifying liquid | 7-8°C | 7-8°C | 7-8°C | 7-8°C | 7-8°C |
| Drying conditions | Drying temperature | 25°C | 15°C | 25°C | 25°C | 20°C |
| | Drying time | 24hr | 10hr | 24hr | 26hr | 18hr |
| | Water content | 2.5% | 5.3% | 2.0% | 2.5% | 4.9% |
| | Water activity | 0.28 | 0.65 | 0.16 | 0.25 | 0.62 |
| | Drying method | Air drying | Air drying | Air drying | Air drying | Air drying |
| | Intermediate layer viscosity upon drying | 180cps | 230cps | 160cps | 180cps | 280cps |

### Immunizing effect confirming test 1

Microparticles containing attenuated TGE viruses obtained in Example 2 at administration of a dose of 10⁷ TCID₅₀ were mixed with the feed and administered to fattened swine of about 3 months of age by natural feeding, twice at intervals of 8 weeks. Blood was collected with a lapse of time from the beginning of administration, and a neutralizing antibody titer against the TGE virus in the serum of swine was measured with a lapse of time. The results are given in Table 2.

**Table 2**

| Test 1 Neutralizing antibody titer in the serum of fattened swine | | | | | | | | | | | | | |
|---|---|---|---|---|---|---|---|---|---|---|---|---|---|
| | Weeks after 1^{st} administration | | | | | | | | | | | | |
| | 0 | 1 | 2 | 3 | 4 | 5 | 6 | 7 | 8 | 9 | 10 | 11 | 12 |
| Immunization | 1^{st} adminis tration | | | | | | | | 2^{nd} adminis tration | | | | |
| Neutralizing antibody titer | <2 | | | 2 | | 8 | | 8 | 8 | 16 | 128 | 128 | 64 |

From the results shown in Table 2, it was confirmed that immunizing effects are recognized even with a single administration of the microparticles containing attenuated TGE viruses obtained in Example 2, and the second administration results in more of an increase in the neutralizing antibody titer.

### Immunizing effect confirming test 2

The microparticles containing attenuated TGE viruses obtained in Example 3 at administration of a dose of 10⁷ TCID₅₀ were mixed with the feed and administered to pregnant swine by natural feeding, five times at intervals of 2 weeks, from before 9 weeks of the expected date of birth. Blood was collected with a lapse of time from the beginning of administration, and a neutralizing antibody titer against the TGE virus in the serum of swine was measured with a lapse of time. In addition, a neutralizing antibody titer in the milk serum after the parturition was also measured. Suckling piglets were divided into an immunized group (breast-feeding group) and a control group (artificial feeding group). On day 3 after the parturition, animals were challenged with a virulent TGE virus strain at administration of a dose of 10⁵ pig infective dose (PID)₅₀/animal by oral administration, and anti-infective effects of the transferred antibodies were investigated. The results are given in Tables 3 to 5.

Test 2 Neutralizing antibody titer in serum of mother swine

**Table 3**

| | Weeks after 1^{st} administration | | | | | |
|---|---|---|---|---|---|---|
| | 0 | 2 | 4 | 6 | 8 | 9 (Parturition) |
| Immunization | 1^{st} administration | 2^{nd} administration | 3^{rd} administration | 4^{th} administration | 5^{th} administration | |
| Neutralizing antibody titer | <2 | <2 | <2 | 256 | 32 | 64 |

**Table 4**

| Test 2 Neutralizing antibody titer in serum and milk serum of mother swine after the parturition | | | | | | | | | | |
|---|---|---|---|---|---|---|---|---|---|---|
| | | Days after parturition | | | | | | | | |
| | | 1 | 2 | 3 | 4 | 5 | 6 | 7 | 8 | 9 |
| Neutralizing antibody titer | Serum | | | 64 | | 128 | | | 2048 | |
| | Milk serum | 2048 | 1024 | 128 | 64 | 32 | 64 | >256 | 64 | 64 |

**Table 5**

| Test 2 Clinical symptoms in suckling piglets after virulent strain challenging | | | | | | | | | |
|---|---|---|---|---|---|---|---|---|---|
| | | Days after viral challenge (days after parturition) | | | | | | | |
| | | 1 | 2 | 3 | 4 | 5 | 6 | 7 | 8 |
| | | (4) | (5) | (6) | (7) | (8) | (9) | (10) | (11) |
| Immunized group | 1 | - | + | ++ | + | - | + | + | Recovered |
| | 2 | + | + | + | + | - | + | + | Recovered |
| | 3 | + | ++ | + | | + | ++ | + | Recovered |
| | 4 | - | - | + | + | - | + | + | Recovered |
| Control | 5 | + | +++ | Dead | | | | | |
| | 6 | ++ | +++ | Dead | | | | | |
| | 7 | ++ | +++ | Dead | | | | | |

| | | | | | | | | | |
|---|---|---|---|---|---|---|---|---|---|
| -: normal stool, +: loose stool, ++: mud-like diarrhea, +++: watery diarrhea | | | | | | | | | |

From the results shown in Tables 3 to 5, immunizing effects in pregnant swine were recognized by several administrations of the microparticles containing attenuated TGE viruses obtained in Example 3. Further, a high neutralizing antibody titer was recognized in the milk serum after the parturition, and symptom-relieving effects were recognized in a challenge test of the virulent TGE virus strain against suckling piglets fed with the milk containing the neutralizing antibody.

### Preservation stability test of microorganism or biological material

The stability of the virus amount (infectivity titer) was examined upon hermetic preservation of the microparticle containing a microorganism or biological materials prepared in Examples 1 to 3 and Comparative Examples 1 and 2 together with a drying agent at 4°C.

The virus amount (infectious titer) was measured according to the following method. First, a quantity of particles (0.253 g) corresponding to 0.1 mL of viruses in terms of a viral liquid volume prior to inclusion of viruses in the microparticle containing a microorganism or biological material was weighed. The weighed particles were mixed with 10 mL of a viral growth medium warmed to 37 to 40°C, and the mixture was homogeneously emulsified and filtered through a filter having a pore diameter of 0.45 to 0.8 µm. Then, the virus amount was measured using cells for virus growth (minipig kidney cells (MPK cells)).

That is, MPK cells were cultured to a monolayer on a 48-well plate. After removal of the culture medium, series of 10-fold dilutions of the test sample were inoculated into 4 wells at a dose of 0.1 mL/well. A half mililiter of a culture medium for cell maintenance was added to each well. The culture was incubated in a CO₂ incubator at 37°C for one week and development of the cytopathic effects (CPE) was observed. The quantity of the infectious virus was calculated by the Karber method and was expressed in terms of log₁₀TCID₅₀/mL (50% tissue culture infectious dose).

The amount of viruses in the core liquid, prior to inclusion of viruses in the particle (pre-granulation), immediately after preparation of the microparticle containing a microorganism or biological material (post-granulation), and after 3 to 21-months of preservation following the preparation of the microparticle (3, 7, 12, and 21 months later) was measured. The results are shown in Table 6 below.

**Table 6**

| | Virus amount (log₁₀TCID₅₀/mL) | | | | | |
|---|---|---|---|---|---|---|
| | Before preparation | Immediately after preparation | 3 months later | 7 months later | 12 months later | 21 months later |
| Ex. 1 | 7.0 | 6.0 | 6.3 | 6.0 | 6.0 | 5.8 |
| Ex. 2 | 7.0 | 5.8 | 6.0 | 6.0 | 5.8 | 5.8 |
| Ex. 3 | 7.0 | 6.5 | 6.0 | 6.0 | 5.5 | 5.5 |
| Comp. Ex. 1 | 7.0 | 6.5 | 5.5 | 4.3 | 3.8 | 3.0 |
| Comp. Ex. 2 | 7.0 | 6.3 | 6.0 | 4.5 | 4.3 | 3.5 |

From the results shown in Table 6, it was found that viruses maintained in the microparticle containing a microorganism or biological materials exhibit excellent preservation stability, with substantially no decrease in the virus amount even upon 21-month preservation at 4°C.

According to the present invention, what is provided is a microparticle containing a microorganism or biological material which is useful as a safe and stable oral vaccine because it is a microparticle containing a microorganism or biological material capable of immunizing an animal.

In the microparticle of the present invention, a core liquid containing a microorganism or biological material is covered by an outermost shell layer composed of an aqueous solution, and an intermediate layer composed of a hydrophobic substance having a viscosity at 50°C of 50 cps or more and a viscosity at 5°C of 500 cps or less, contact of a microorganism or biological material with an organic solvent or exposure thereof to high temperatures occurring in conventional coating methods is avoided, and inactivation of a microorganism or biological material can be reduced. Therefore, the present invention is very effective particularly when an attenuated pathogen is used.

Further, a production process is simple and continuous production is also possible, thus achieving high productivity and low production costs.

The microparticle containing a microorganism or biological material in the present invention has a total water content of 3% or less and a water activity value of 0.5 or less, so inactivation of a microorganism or biological material because of water can be significantly reduced, and therefore preservation properties during commercial distribution of products can be maintained for a relatively long period of time.

While preferred embodiments of the invention have been described and illustrated above, it should be understood that these are exemplary of the invention and are not to be considered as limiting. Additions, omissions, substitutions, and other modifications can be made without departing from the spirit or scope of the present invention. Accordingly, the invention is not to be considered as being limited by the foregoing description, and is only limited by the scope of the appended claims.

## Claims

1. A microparticle containing a microorganism or biological material having a three-layered structure composed of an outermost shell layer, an intermediate layer, and a core, wherein
the outermost shell layer is composed of a composition containing gelatin and natural gum, an aqueous solution of the composition has a gelling temperature of 60°C or lower,
the intermediate layer is composed of a hydrophobic substance having a viscosity at 40°C of 250 cps or less and a viscosity at 5°C of 300 cps or more,
the core is composed of a composition containing a microorganism or biological material, and
a water content of the whole particle is 5% or less and a water activity is 0.5 or less.

2. The microparticle containing a microorganism or biological material according to claim 1, which has a network structure which is formed by the reaction of divalent cations with natural gum contained in the outermost shell layer.

3. The microparticle containing a microorganism or biological material according to claim 1 or 2, wherein the hydrophobic substance constituting the intermediate layer is a mixture of lecithin and lipids.

4. A process for producing a microparticle containing a microorganism or biological material, comprising:
forming a seamless capsule precursor having a three-layered structure composed of an outermost shell layer, an intermediate layer, and a core liquid, by a method of extrusion into a solidifying liquid using a concentric triple nozzle, and
drying the seamless capsule precursor to a water content of the whole particle of 5% or less and a water activity of 0.5 or less, wherein
the outermost shell layer is composed of a composition containing gelatin and natural gum, an aqueous solution of the composition has a gelling temperature of 60°C or less,
the intermediate layer is composed of a hydrophobic substance having a viscosity at 40°C of 250 cps or less and a viscosity at 5°C of 300 cps or more, and
the core liquid is composed of an aqueous liquid containing a microorganism or biological material.

5. The process for producing a microparticle containing a microorganism or biological material, according to claim 4, comprising dipping the seamless capsule precursor extruded from the concentric triple nozzle into a liquid containing divalent cations, prior to drying.

6. The process for producing a microparticle containing a microorganism or biological material, according to claim 4 or 5, wherein drying of the seamless capsule precursor is carried out at a temperature equal to or higher than the temperature where the intermediate layer has a viscosity of 250 cps or less and at a drying temperature equal to or lower than a gelling temperature of an aqueous solution of the composition constituting the outermost shell layer.

7. The process for producing a microparticle containing a microorganism or biological material, according to any one of claims 4 to 6, wherein a center tube and/or an intermediate tube of the concentric triple nozzle have thermal insulation properties.
